# EUROPEAN PATENT SPECIFICATION

(11) **EP 1 959 840 B1**
(45) Date of publication and mention of the grant of the patent: **08.02.2012**
(21) Application number: 06846176.3
(22) Date of filing: 27.10.2006
(51) Int. Cl.: A61B 17/06, A61B 17/04, A61B 17/00, A61B 17/064

(54) **DELIVERY SYSTEM FOR A BARBED FASTENER**
AUSGABESYSTEM FÜR EINEN VERSCHLUSS MIT WIDERHAKEN
SYSTEME DE DEPLOIEMENT D'UNE AGRAFE BARBELEE

(30) Priority: 31.10.2005 US 731826 P
(43) Date of publication of application: 27.08.2008
(73) Proprietor: ETHICON, INC., Somerville, New Jersey 08876 (US)
(72) Inventor: COLLIER, John, Franklin Lakes, NJ 07417 (US); RUDNICK, James, Mahwah, NJ 07430 (US); LINDH, SR., David C., Flemington, NJ 08822 (US); SLATER-TOMKO, Andrea, Easton, PA 18040 (US)
(74) Representative: James, Anthony Christopher W.P.
(86) International application number: PCT/US2006/060307
(87) International publication number: WO 2007/053812

(56) References cited:
- EP-A- 0 632 999
- WO-A-97/18762
- WO-A-2005/096955
- WO-A2-2004/030517
- BE-A6- 1 014 364
- US-A- 3 716 058
- US-A- 5 342 376
- US-A1- 2003 130 694
- US-A1- 2005 203 576

## Description

### Field of the Invention

The present invention relates generally to the field of surgical sutures, and more particularly to a delivery system for a surgical suture assembly having a barbed configuration.

### Background

It is well known to use barbed fasteners to treat facial ptosis or other types of facial sagging. Surgeons generally use two methods. In the first, a surgeon uses a hollow needle to create a first, entrance puncture in the skin and, after advancing the needle through tissue, creates a second, exit puncture in the skin distance from the first puncture along a predetermined pulling vector. The barbed fastener is then advanced through the hollow needle and grasped at the exit puncture, and the needle is withdrawn. The surgeon then manipulates the tissue and the exposed barbed suture to create the desired effect; i.e., lift the sagging skin. Finally, the exposed ends of the suture are cut below the surface of the skin. In some cases, a dimple in the skin can form at one or both of the exit points, and the surgeon releases some of the barbs near the surface to eliminate any dimple.

The second method involves the use of straight needles that are attached to opposite ends of the barbed fastener, and creates three punctures. First, the surgeon creates an entrance puncture at the approximate midpoint of the area to be treated, and advances the needle along with one end of the barbed fastener until the needle exits the skin some distance away at a location along a predetermined pulling vector. Next, the surgeon inserts a second needle, attached to the opposite end of the barbed fastener, through the initial midpoint puncture, and advances it in the direction opposite to that of the first needle through the tissue. The second needle is pushed through the tissue until it exits, creating a third puncture in the skin. The surgeon then manipulates the tissue and the exposed barbs on either end of the fastener to create the desired effect. Finally, the exposed ends of the suture are cut below the surface of the skin.

Prior art devices for delivering barbed fasteners have been disclosed, for example, in U.S. Patent Nos. 3,716,058 (Tanner) and 5,342,376 (Ruff). The Tanner device teaches that the barbed fastener needs to be inserted in two steps. First, one barbed end of the fastener is carried by the needle in a notch and when located at the desired location, the other barbed end of the fastener is loaded into the needle and placed at a second desired location. Ruff describes a barbed fastener insertion device that houses the barbs except for perhaps one barb that may extend from the distal end to prevent the fastener from being pulled through the body of the insertion device. The fastener of Ruff includes a pointed end that facilitates gentry of the device into tissue. Ruff discloses passing the pointed end of the fastener and the insertion device into and out of the tissue, thereby creating two punctures. Among the disadvantages of the Tanner and Ruff devices, they do not allow for intimate contact during insertion between a plurality of barbs and the tissue within which the barbs will be anchored. Further Tanner and Ruff do not describe a method in which a barbed fastener can be applied within tissue in a single pass while creating only one puncture.

WO-A-2004030517 describes method of fastening tissue using a barbed suture having sharp pointed ends Each end of the suture includes barbs that permit movement in an opposing direction to the barbs on the other end of the suture.

WO-A-1718762 describes a surgical fastening system using a short collagen fastener having barbs on one end and an introducer tool for applying the fastener.

US-A-3716058 describes a surgical suture comprising a relatively short section of rigid or flexible suture material with a return barb on both ends.

Thus, it would be desirable to provide a barbed fastener delivery device that is configured to dissect tissue and permit a plurality of barbs to contact the tissue through which it is passed during insertion. Further, is desirable to provide a delivery device that permits a barbed fastener to be applied within tissue in a single pass while creating only one puncture. Minimization of punctures prevents dimples from being created when barbs engage tissue too near the tissue surface.

### Summary of the Invention

The present invention provides a fastener delivery device comprising: a fastener having a body, a longitudinal axis and a plurality of barbs extending from the body along the longitudinal axis; and a cannula comprising: a proximal end, a distal end and a distal tip wherein the proximal end and the distal end of said cannula are each configured to receive a portion of the fastener, and the cannula has an opening that expose at least two of the plurality of barbs when at least a portion of the fastener is disposed within the cannula, wherein the opening is a slot extending from the distal tip toward the proximal end.

The device suitably includes a fastener having a body, a longitudinal axis, a first set of barbs extending from the distal end of the body and a second set of barbs extending from the distal end of the body offset axially from the first set of barbs. The cannula suitably has a first opening and a second opening offset radially from the first opening, the first opening being configured to expose at least two barbs of the first set of barbs and the second opening being configured to expose at least two barbs of the second set of barbs when at least a portion of the fastener is disposed within the cannula.

In use, an opening is created in tissue. The distal end of the listener delivery device is passed into tissue located at a first position, with the at least two of the plurality of barbs being exposed to the tissue. At least a portion of the at least two of the plurality of barbs is disposed within the tissue to displace the tissue from the first position to a second position. Finally, the cannula is removed while the barbs are disposed within the tissue.

These and other objects, features and advantages of the present invention will be apparent from the hollowing detailed description of illustrative; embodiments thereof, which is to be read in connection with the accompanying drawings.

### Brief Description of the Drawings

Figs. Ia, Ib and Ie are perspective views of different embodiments of barbed fasteners used with the present disclosure;
Fig. 2 is a perspective view illustrating one embodiment of the delivery system having a sharpened distal end and a distal opening;
Fig. 3 is a side cross-sectional view of Fig. 2;
Fig. 4 is an end view of Fig. 2;
Fig. 5 is a perspective view illustrating an alternate embodiment of the delivery system having a sharpened distal end and a slot;
Fig. 6 is a side cross-sectional view of Fig. 5;
Fig. 7 is an end view of Fig. 5;
Fig. 8a-8c is a side view of an alternate embodiment of the invention;
Figs. 9a and 9b are perspective views of another alternate embodiment having dissecting tip formed of at least two portions in a first configuration and second configuration, respectively;
Fig. 10 is a perspective view of another alternate embodiment having a dissecting tip formed of at least three portions;
Fig. 11 is cross-sectional perspective view of a distal portion of a fastener delivery system of the present disclosure depicting an exit opening;
Figs. 12a-12d illustrate steps of one method of using the fastener delivery device of the present disclosure; and
Figs. 13a-13c illustrate steps of one method of using the fastener delivery device of the present disclosure to reduce nasal fluid flow resistance.

### Detailed Description

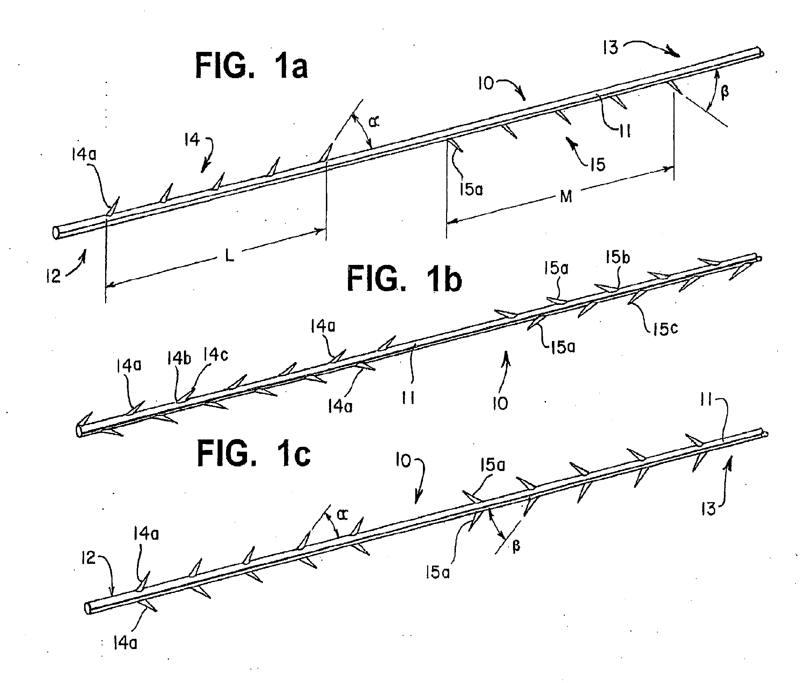

Referring now to Figs. 1a through 1c, different types of barbed fasteners are depicted as examples of those types of fasteners that may be deployed by the fastener delivery of the invention. One example, depicted in Fig. 1a, is a barbed fastener 10 having a body 11, a distal portion 12 and a proximal portion 13. Fastener 10 may have barbs 14a, 15a that extend along its entire length or along only a portion or portions of its length. In a preferred embodiment, a first set or plurality of barbs 14 and a second set or plurality of barbs 15 each may include a plurality of barbs 14a, 15a, that preferably number at least three, and more preferably at least five. First set of barbs 14 extend from distal portion 12 of body 11 substantially along a first predetermined length L in a first direction at an angle a that is less than 90 degrees relative to the longitudinal axis. Second set of barbs 15 extend from proximal portion 13 of body 11 substantially along a second predetermined length M in a second direction at an angle β that is greater than 90 degrees but less than 180 degrees relative to the longitudinal axis.

As shown in Fig. 1b, each barb 14a, 15a extends from body 11 at a base 14b, 15b to a free end 14c, 15c. Fastener 10 may include a predetermined unbarbed length between first set of barbs 14 and second set of barbs 15. First set of barbs 14 may be offset radially from the second set of barbs 15, as is shown in Fig. 1a, preferably by approximately 180 degrees. Alternatively, fastener 10 may include barbs 14a that extend radially from different points along, the circumference of body 11 in a staggered manner, as shown in-Fig. 1b, or in an aligned manner, i.e., extending from approximately the same position along the length of body 11, but offset radially by some angle, as shown in Fig. 1c. fastener 10 may include a body that has a circular, ovate, elliptical or triangular cross section, for example. In one embodiment, fastener 10 does not have any barbs along a length of proximal end 11.

Fastener 10 may be made of any suitable nonabsorbable material such as polypropylene, or any suitable absorbable material such as poly(glycolide-lactide) or poly(glycolide-ε-caprolactone). Alternatively, fastener 10 could be formed from a shape memory polymer, such as a polyurethane-based polymer, so as to facilitate deployment of the barbs after exposure to the transition temperature of the shaped memory polymer. The suture size may vary but preferably ranges from size 3-0 to size 1, most preferably size 2-0 to size 0.

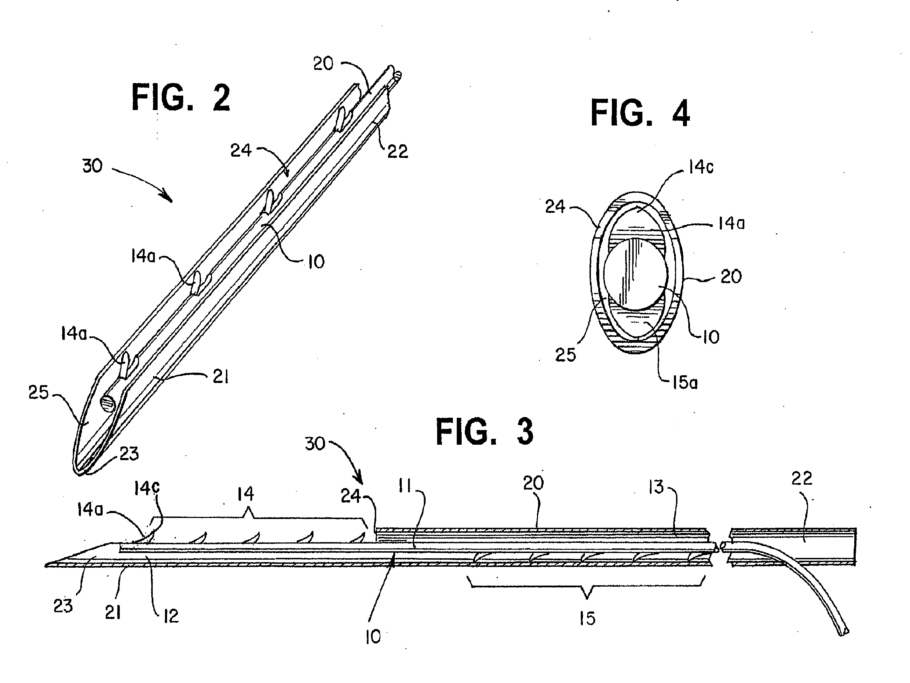

Referring to Fig. 2, a first embodiment of a fastener delivery device 30 is depicted that includes a cannula 20 and a bared fastener 10 at least partially disposed within a lumen 25 of cannula 20. Lumen 25 of cannula 20 is preferably coaxial with the longitudinal axis that intersects the center of the cannula cross section, but may be offset from such longitudinal axis. In this case, fastener 10 has a first plurality of barbs 14 expending from distal end 12 of body 1 and a second plurality of barbs 15 extending from proximal end 13 of body 11 that are offset radially approximately 180 degrees from the first plurality of barbs. Cannula 20 has a distal end 21 and a proximal end 22, a distal tip 23 and an opening 24 that extends from the distal tip in a proximal direction for a predetermined length. Distal tip 23 is preferably sharp, but must at least be configure so as to dissect tissue and thereby create a pathway through which cannula 20 may pass. In one embodiment, distal tip 23 is ground at a 45 degree angle to a sharpened point.

Opening 24 may be formed by removing a portion of cannula 20 to form a slot. Opening 24 is configured to be of a length that permits at least two barbs 14a to be exposed to tissue through which cannula 20 is passed. That is, as is best seen in Fig. 3, the free ends of at least two barbs 14a extend beyond the cannula wall such that the free ends 14c are exposed to tissue when fastener delivery device 30 is in use. Opening 24 is configured to have a width that is less than the diameter of body 11 of fastener 10 so as to prevent fastener 10 from being inadvertently released from cannula 40.

The cross section of cannula 20 may be of any shape, such as circular, ovate or triangular, for example, but preferably is configured to have a portion that provides space to hold barbs in a deflected position; i.e., a position in which the barb extends from the body at an angle relative to the longitudinal axis. One example is shown in Fig. 4, where the cross section of cannula 20 is ovate to better conform to a fastener having a second set of barbs 15 that are 180 degrees radially offset from the first set of barbs 14. In that case, the second set of barbs 15 can be disposed within cannula 20 without being constrained in a flattened position. If barbs 15 are constrained for a period of time, they may retain a memory of that position and may not extend to a configuration that is better able to bite and hold tissue.

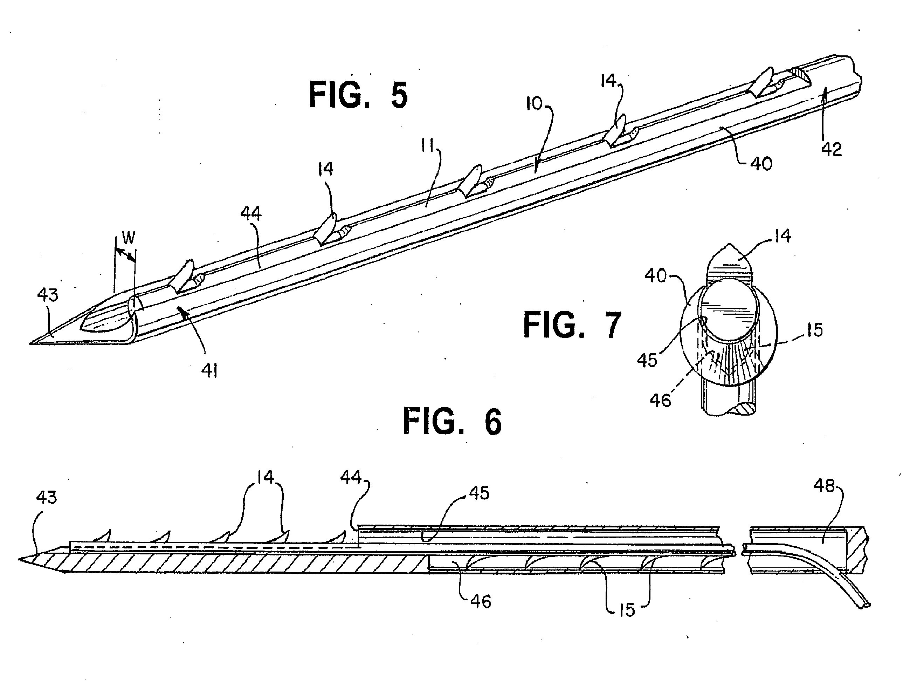

Referring to Figs. 5-7, a second embodiment of a fastener delivery device is depicted that includes a cannula 40 configured to at least partially receive a barbed fastener 10 within a passageway 45 of cannula 40. Cannula 40 has a distal end 41 and a proximal end 42, a distal tip 43 and an opening 44 that extends from the distal tip in a proximal direction for a predetermined length. As with the first embodiment, opening 44 is configured to have a width W that is less than the diameter of body 11 of fastener 10 so as to prevent fastener 10 from being inadvertently released from cannula 40. Distal tip 43 is preferably sharp, but is at least configured to dissect tissue and thereby create a pathway through which cannula 40 may pass.

Opening 44 is configured to be of a length that permits at least two barbs to be exposed within opening 44. Cannula 40 may be molded to form an opening or slot 44.Fig. 5 shows opening 44 extending along the entire length of the depicted portion of cannula 40, but it is understood that opening 44 can be so configured, but may also be of a shorter predetermined length. As best seen in Fig. 7, in this embodiment, passageway 45 is offset from a longitudinal axis that intersects with the midpoint of the cross section. Further, cannula 40 includes a key 46 that extends for a predetermined distance and communicates with passageway 45. Key 46 is configured to retain a plurality of barbs that extend from the distal end of the fastener in a non-flattened configuration such that second set of barbs 15 can be disposed within cannula 40 without being constrained in a flattened position. As a result, barbs 15 are more likely to retain a configuration that is better able to bite and hold tissue as they will not be constrained in a non-extended position within cannula 40.

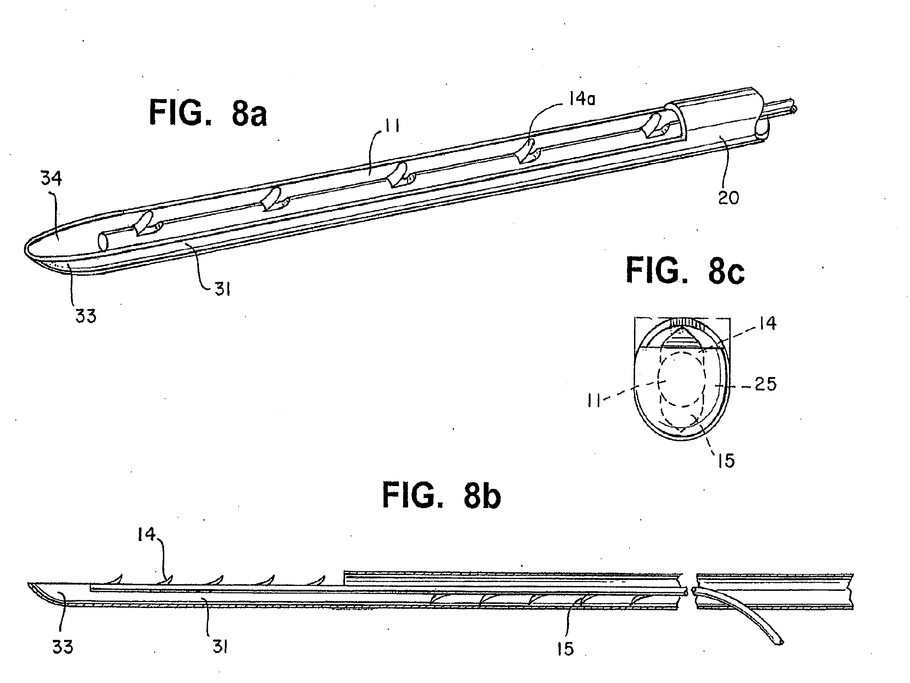

Referring to Figs. 8a-8c, an alternative embodiment of the distal end of cannula 20 is shown and referred to as reference numeral 31. Distal end 31 is configured similarly to an epidural needle in that it has an opening 34 oriented on one side of cannula 20; i.e., substantially parallel to the longitudinal axis of cannula 20. Distal tip 33 of this embodiment serves to pierce tissue T. Opening 34 communicates with lumen 25. Thus, when fastener 10 is separated from cannula 20, fastener 10 exits lumen 25 and opening 34 on one side of cannula 20.

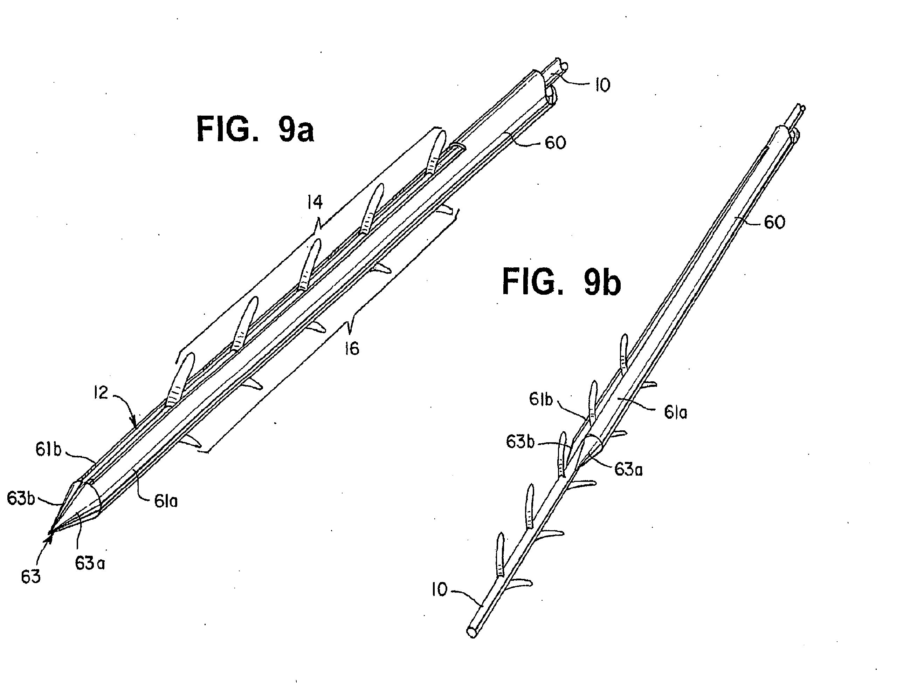

Referring to Figs. 9a and 9b, the distal end of an embodiment of a fastener delivery device is depicted in a first configuration and a second configuration, respectively. In this embodiment, distal end 12 of cannula 60 is split, preferably along the longitudinal axis, to form at least a first distal portion 61a and a second distal portion 61b. Preferably, the split is widened at two locations along the longitudinal axis to form two openings to expose first set of barbs 14 and second set of barbs 16 to tissue when fastener 10 is at least partially disposed within cannula 60. The distal most end 63, includes a sharpened distal tip formed, in this embodiment, of sharpened portions 63a and 63b. Portions 61a and 61b may include mating elements, such as a male and female component (not shown), to assist in retaining portions 61a and 61b in the mated first configuration. As depicted in Fig. 9b, and described in more detail below, as cannula 60 is moved proximally relative to fastener 10, portions 61a and 61b flex to permit cannula 60 and fastener 10 to be separated and fastener 10 to travel past flexed portions 61a and 61b to a position distal to distal end 63.

It is understood that while Figs. 9a and 9b depict an embodiment configured to retain a double-barbed distal set of barbs and therefore form two openings to expose each set of barbs, the invention contemplates retaining a single set of barbs or more than two sets of barbs within the lumen of the cannula, with one or more sets of barbs being exposed to tissue when the fastener delivery device is in use. It is also understood, that while the barbs are formed along a longitudinal direction, the plurality of barbs may be formed in an pattern about body 11 of fastener 10.

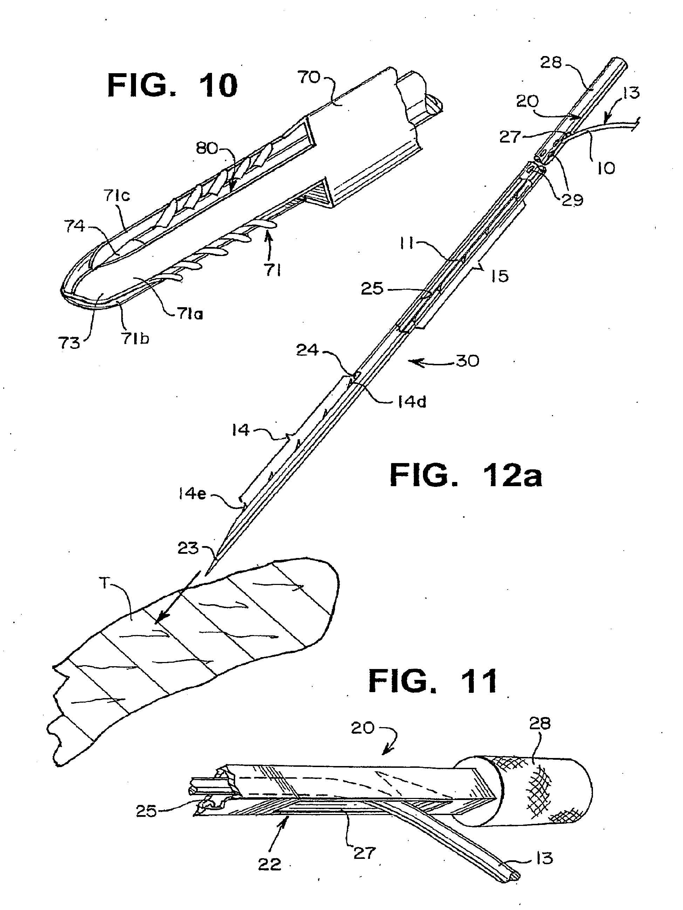

Referring to Fig. 10, a fastener insertion device including a triangular barbed fastener 80 and a triangular cannula 70, are shown. Similar to the embodiment depicted in Figs. 9a and 9b, this embodiment includes a distal end 71 that forms openings within which the barbs of the triangular fastener 80 are exposed during use. Distal end 71 is formed with three arms 71a, 71b and 71c that extend from a proximal portion of cannula 70 and bend radially inward at a distal most location to form a distal tip 73. As with the prior embodiment, as cannula 70 is moved proximally relative to fastener 80, arms 71a, 71b and 71c flex to permit fastener 80 and cannula 70 to be separated as fastener 80 moves distally beyond the distal end of cannula 70.

Fig. 11 depicts proximal end 22 of fastener delivery device 20, which has an exit opening 27 that permits the proximal end 13 of fastener 10 to exit from lumen 25 of cannula 20. Proximal end 22 also includes a handle 28 for the user to maneuver fastener delivery device 20. Exit opening 27 may be oriented with respect to the opening 24 so as to provide an external indicator of the orientation of the distal set of barbs relative to the patient's tissue. For example, exit opening 27 may be oriented at the same position along the circumference of cannula 20 as opening 24 or may be 180 degrees out relative to the position of opening 24. The user can then position cannula 20 knowing the position of the barbs when the distal end of cannula 20 is disposed within tissue.

In an alternative embodiment, fastener 10 can be contained within cannula 20 such that it would not include an exit opening 27. In this embodiment, distal portion 22 of cannula 20 would be configured to be of a length that would contain the proximal end of fastener 10. In use, when the distal set of barbs 14 are engaged within tissue T, cannula 20 would simply be withdrawn leaving fastener 10 in place.

Figs. 12a-12d depict the use of one embodiment of fastener insertion device 30. Fig. 12a depicts fastener 10 partly disposed within lumen 25 of device 30. In this particular embodiment, fastener 10 has a distal set of barbs 14 extending longitudinally from a distal point to a predetermined proximal point. Fastener 10 is configured such that when proximal-most barb 14d is exposed within opening 24, distal-most barb 14e is located proximal of distal tip 23 of cannula 20. In this way, the distal plurality of barbs 14 are contained within the opening 24 of cannula 20. This configuration is not required, but is preferred. For example, it is contemplated that not all of the distal barbs need to be exposed within opening 24. Fastener 10 also includes a proximal set of barbs 15 that are disposed within lumen 25 of cannula 20. As is shown in Fig. 12a, barbs 15 preferably extend at an angle with respect to the longitudinal axis of body 11 of fastener 10. Proximal end 13 of fastener 10 extends out of side exit 27 of cannula 20. Markers 29 are located either on cannula 20 or fastener 10 to indicate where the proximal set of barbs 15 are located or to indicate how much of the cannula 10 is disposed within tissue T.

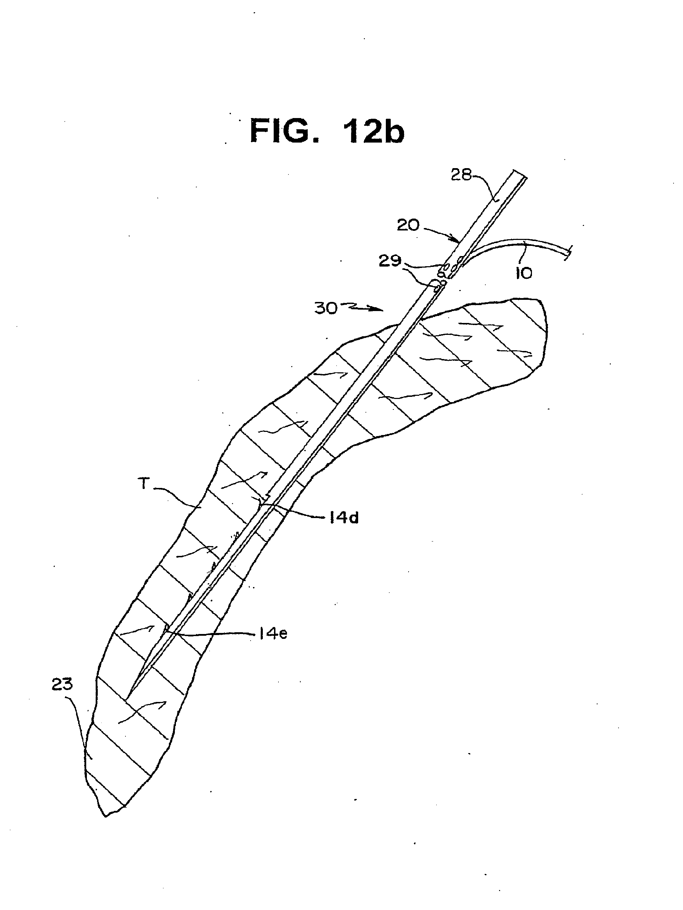

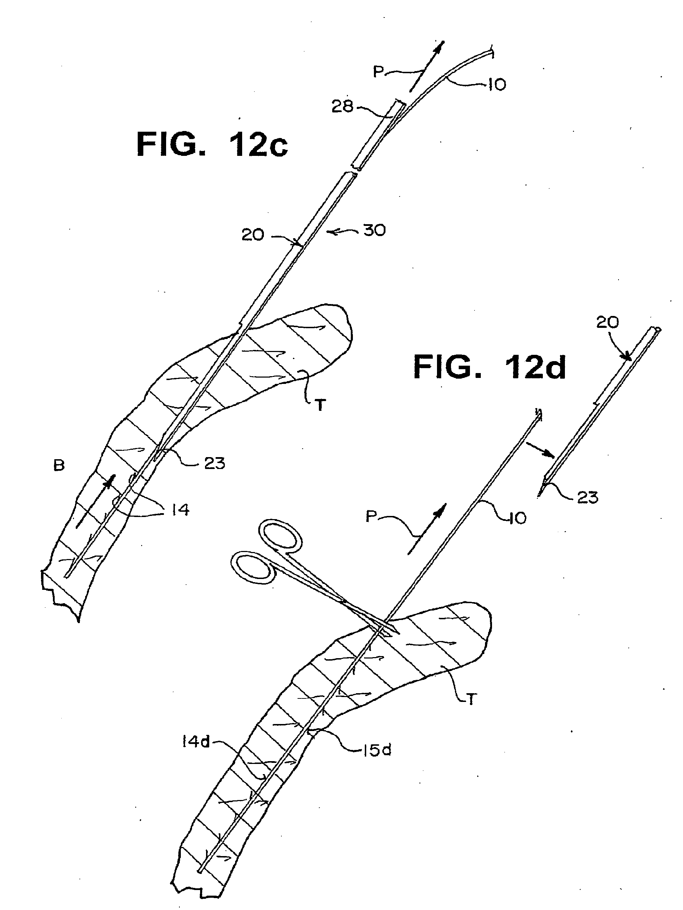

As shown in Fig. 12a, the user grasps fastener delivery device 30 with handle 28 and positions sharpened distal end 23 proximate tissue T. Referring to Fig. 12b, the user then pierces tissue T with distal end 23, which dissects tissue to provide a pathway for cannula 20. While cannula 20 is passed through tissue, the plurality of distal barbs are exposed to tissue T and are deflected back toward body 11 of fastener 10 by tissue T to permit fastener 10 and cannula 20 to pass through tissue T without undue resistance. When cannula 20 is passed to a point just distal of that shown in Fig. 12b, where marker 29 indicates that the entire set of proximal barbs 15 is disposed within tissue T, the user can then withdraw cannula 20 by withdrawing cannula 20 in a proximal direction as indicated by arrow P of Fig. 12c. When the user pulls on handle 28 of cannula 20, free ends 14c of distal barbs 14 (exposed within opening 24) become embedded in tissue T, thereby preventing fastener 10 from being withdrawn with cannula 20. Because the distal set of barbs 14 are exposed to tissue T during insertion of cannula 20, barbs 14 immediately bite or engage tissue T when the user pulls proximally on cannula 20 as indicated by arrow B of Fig. 12c, thereby improving the reliability of the positioning of fastener 10.

The user continues to withdraw cannula 20, which has the effect of paying proximal end 13 of fastener 10 through exit opening 27 of cannula 20 until cannula 20 is separated from fastener 10. At this stage, the user can pull proximally on proximal end 13 of fastener 10, as is shown by arrow P in Fig 12d, and optionally massage tissue such that the proximal set of barbs 15 bite into tissue T at a position more proximal than barbs 15 would otherwise engage. This step has the effect of displacing tissue grabbed by proximal barbs 15 toward distal barbs 14 and creates tension in body 11 of fastener 10 at least between proximal-most distal barb 14d and distal-most proximal barb 15d and perhaps over a longer length of body 11 having plurality of barbs 14, 15. Finally, as is also indicated in Fig. 12d, fastener 10 can be cut at a location just below the surface of tissue T.

In this way, the proposed method provides the advantage of not requiring the creation of an exit wound, as the distal set of barbs 14 engage and hold tissue T at the point where cannula 20 is removed. The elimination of the exit puncture also results in eliminating dimples created when barbs engage tissue too near the tissue surface. The fastener delivery device 30 and single puncture technique are beneficial for the treatment of facial ptosis, since it allows the use of more supple fasteners and eliminates the exit puncture wound. The inventive device also provides the user the ability to expose barbs 14 without locating the distal end of fastener 10 beyond the distal tip 23 of cannula 20. In this way, the distal end of fastener 10 is shielded from any force applied during insertion and thus will not buckle when applied to tissue.

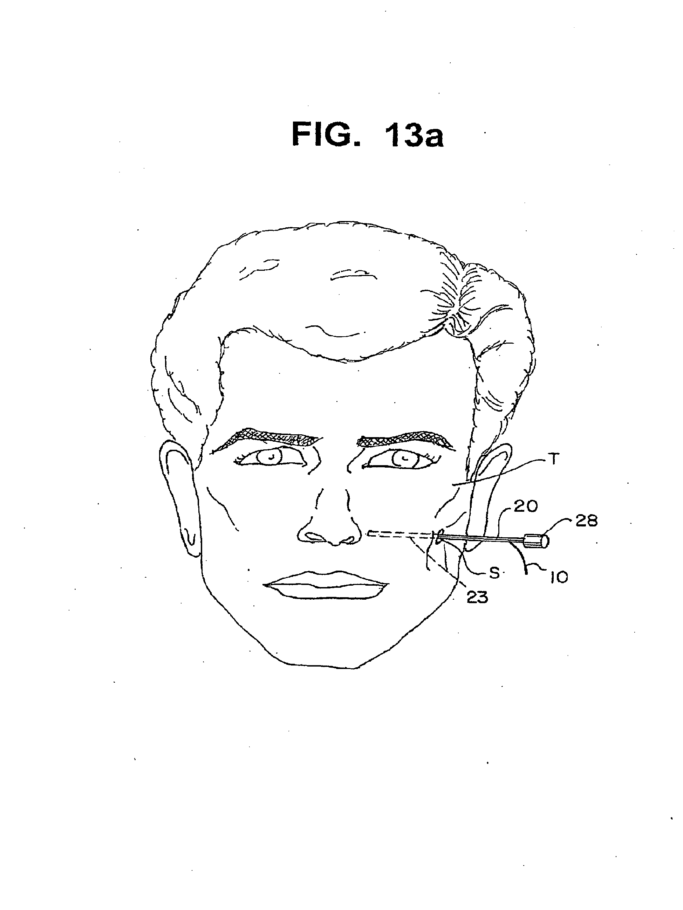

One particular method that fastener delivery device 30 can be used to advantage is to reduce nasal fluid flow resistance as depicted in Figs. 13a-13c. In such a method, a fastener delivery device 30 having a fastener 10 and a cannula 20 is provided, as shown in Fig. 13a. The user pierces tissue T to create puncture S with distal end 23 transbucally into the soft tissue at a position near the zygomatic bone below the eye and in a medial direction toward the nose, preferably the transverse part of the nasalis muscle. Distal end 23 dissects tissue to provide a pathway for cannula 20. The distal end of the device is passed through the soft tissue layer, to a tissue plane superior to the superficial musculoaponeurotic system (SMAS) in the subcutaneous tissue to a position near the upper region of the nostril. This position is shown in Fig. 13a. While cannula 20 is passed through tissue, the plurality of distal barbs are exposed to tissue T and are deflected back toward body 11 of fastener 10 by tissue T to permit fastener 10 and cannula 20 to pass through tissue T without undue resistance.

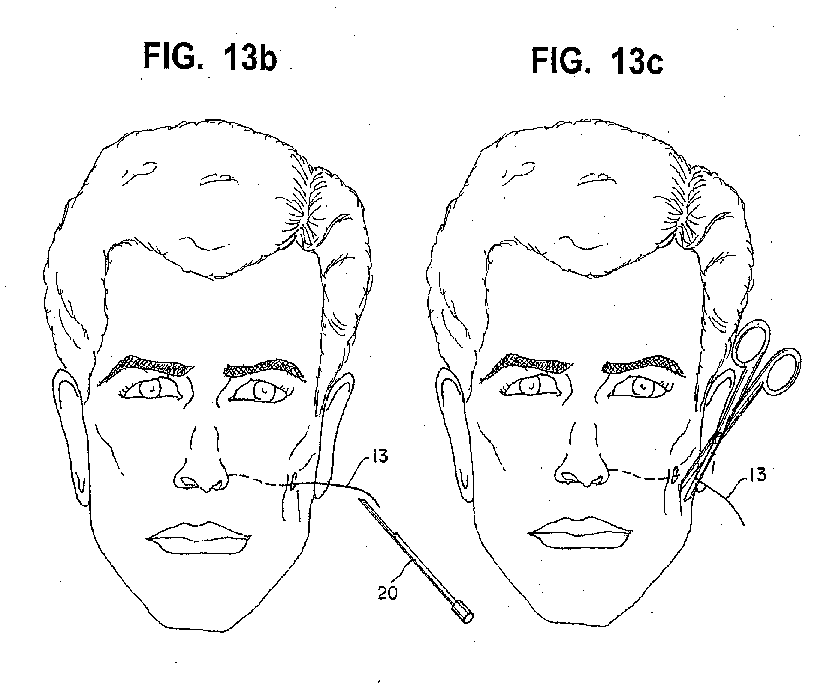

Upon reaching the position depicted in Fig. 13a, cannula 20 is withdrawn, as depicted in Fig. 13b, and the distal barbs engage tissue to prevent fastener 10 from being pulled out with cannula 20. The surgeon may then grasp the proximal, exposed end of the fastener 13, and massage the tissue (skin) located superior of that portion of fastener 10 that is disposed within the tissue, in a lateral direction, so as to move the tissue laterally away from the nostril. Alternatively, proximal end 13 of fastener 10 is pulled proximally to a position within the tissue so as to engage the tissue with the proximal set of barbs 15 to displace tissue near the nostril. Either of these actions will cause tissue near the nostril to be displaced and change the geometry of the nasal passage in such a way as to create a larger effective opening (dilate the nasal passage) for improved fluid flow. Upon achieving the desired effect, the suture is cut just below the skin to remove the excess material as shown in Fig. 13c.

Optionally, prior to the method, the patient's nasal passage fluid flow may be measured and compared to the fluid flow just prior to the step of cutting the proximal end of the fastener. In this way, the surgeon may determine whether the geometry of the nasal passage has been effectively altered to improve fluid flow. If it has not, the surgeon may reorient the fastener to displace additional tissue.

### Exposed Barb Test

A test was conducted to determine how many barbs were required to be exposed to tissue to reliably bite and hold tissue at a desired location. The test method utilized a cannula having a 0.79 mm (0.031 inch) inner diameter and a 0.91 mm (0.036 inch) outer diameter and the distal tip having a 45° ground end to serve as the sharpened point for tissue penetration. The cannula was machined to create an opening or channel that extended from tip proximally approximately 23 mm (0.9 inches). Size 0 polypropylene suture having two rows of seven barbs each positioned 180° apart along the circumference and offset was disposed within the cannula.

Different strands of suture were positioned such that varying numbers of distally located barbs were disposed within the opening and therefore were expose to tissue as the cannula was passed through the tissue. First one barb was exposed, then two barbs, and with each successive iteration an additional barb was exposed. The test was repeated multiple times with one barb and two barbs to gain confidence in the findings. The cannula was inserted into tissue and then removed to determine whether the exposed barb or barbs would engage the tissue at the point where the cannula was removed and then hold the tissue.

Referring to Table 1, the results demonstrate that more than one exposed barb is needed in the channeled insertion device to reliably engage the barbs in tissue. While two of the one-barb tests engaged tissue, they did so only after the cannula was partly removed from the tissue, and thus did not immediately engage at the deepest point of insertion. This performance is not acceptable as the barbs may not engage in the correct location, and therefore, may not be effective in pulling the tissue back at the desired location. The tests that exposed at least two barbs easily and immediately engaged in tissue upon removal of the insertion device.

**Table 1**

| No. of exposed barbs | Test # | Hold/No Hold | Comment |
|---|---|---|---|
| 7 | 1 | Held | |
| 6 | 1 | Held | |
| 5 | 1 | Held | |
| 4 | 1 | Held | |
| 3 | 1 | Held | |
| 2 | 1 | Held | |
| | 2 | Held | |
| 1 | 1 | Held | Slipped and then grabbed tissue during extraction |
| | 2 | No Hold | |
| | 3 | No Hold | |
| | 1 | Held | Slipped and then grabbed tissue during extraction |

Although illustrative embodiments of the present invention have been described herein with reference to the accompanying drawings, it is to be understood that the invention is not limited to those precise embodiments and that various other changes and modifications may be effected herein by one skilled in the art without departing from the scope of the invention as defined in the accompanying claims.

## Claims

1. A fastener delivery device, comprising:
a fastener (10) having a body (11), a longitudinal axis and a plurality of barbs (14a,15a) extending from the body along the longitudinal axis; and
a cannula (20,40,60), wherein
the cannula comprises: a proximal end (22,42), a distal end (21,41) and a distal tip (23,43,63) wherein the proximal end and the distal end of said cannula are each configured to receive a portion of the fastener, and the cannula has an opening (24,44) that exposes at least two of the plurality of barbs (14a) when at least a portion of the fastener is disposed within the cannula, **characterised in that** the opening is a slot extending from the distal tip toward the proximal end.

2. The fastener delivery device of claim 1, wherein the cannula includes a sharpened distal tip (23,43,63).

3. The fastener delivery device of claim 1, wherein the fastener comprises a first plurality of barbs (14) extending from the distal end of the body and a second plurality of barbs (15) offset longitudinally and radially from the first set of barbs.

4. The fastener delivery device of claim 3, wherein the body has a length having no barbs between the first plurality of barbs (14) and the second plurality of barbs (15).

5. The fastener delivery device of claim 3, wherein the first plurality of barbs (14) extend outwardly from the body (11) in a first direction less than 90 degrees from the longitudinal axis and the second plurality of barbs (15) extend outwardly from the body in a second direction that is greater than 90 degrees and less than 180 degrees from the longitudinal axis.

6. The fastener delivery device according to claim 5, wherein the tips of the first plurality of barbs (14) and the tips of the second plurality of barbs (15) are directed toward one another.

7. A fastener delivery device according to claim 1, wherein:
said fastener (10) has a first set of barbs (14) extending from the distal end of the body and a second set of barbs (15) extending from the distal end of the body offset axially from the first set of barbs; and
said cannula (20,40,60) is configured to receive at least a portion of the fastener (10) and retain a plurality of barbs (15) such that the plurality of barbs extend from the body (11) at an angle from the longitudinal axis.

## Patentansprüche

1. Vorrichtung zur Abgabe von Befestigungselementen, die Folgendes umfasst:
ein Befestigungselement (10) mit einem Körper (11), einer Längsachse und einer Vielzahl von Widerhaken (14a, 15a), die sich vom Körper entlang der Längsachse erstrecken; und
eine Kanüle (20, 40, 60), worin die Kanüle Folgendes umfasst: ein proximales Ende (22, 42), ein distales Ende (21, 41) und eine distale Spitze (23, 43, 63), worin das proximale Ende und das distale Ende der Kanüle jeweils so konfiguriert sind, dass sie einen Teil des Befestigungselements aufnehmen und die Kanüle eine Öffnung (24, 44) aufweist, die zumindest zwei der Vielzahl von Widerhaken (14a) freilegt, wenn zumindest ein Teil des Befestigungselements in der Kanüle angeordnet ist, **dadurch gekennzeichnet, dass** die Öffnung ein Schlitz ist, der sich von der distalen Spitze zum proximalen Ende hin erstreckt.

2. Vorrichtung zur Abgabe von Befestigungselementen nach Anspruch 1, worin die Kanüle eine angespitzte distale Spitze (23, 43, 63) aufweist.

3. Vorrichtung zur Abgabe von Befestigungselementen nach Anspruch 1, worin das Befestigungselement eine erste Vielzahl von Widerhaken (14), die sich vom distalen Ende des Körpers aus erstrecken, und eine zweite Vielzahl von Widerhaken (15) aufweist, die in Längsrichtung und radial vom ersten Widerhakensatz versetzt sind.

4. Vorrichtung zur Abgabe von Befestigungselementen nach Anspruch 3, worin der Körper eine Strecke ohne Widerhaken zwischen der ersten Vielzahl von Widerhaken (14) und der zweiten Vielzahl von Widerhaken (15) aufweist.

5. Vorrichtung zur Abgabe von Befestigungselementen nach Anspruch 3, worin sich die erste Vielzahl von Widerhaken (14) vom Körper (11) in einer ersten Richtung weniger als 90 Grad von der Längsachse nach außen erstreckt und sich die zweite Vielzahl von Widerhaken (15) vom Körper in einer zweiten Richtung, die mehr als 90 Grad und weniger als 180 Grad von der Längsachse entfernt ist, nach außen erstreckt.

6. Vorrichtung zur Abgabe von Befestigungselementen nach Anspruch 5, worin die Spitzen der ersten Vielzahl von Widerhaken (14) und die Spitzen der zweiten Vielzahl von Widerhaken (15) aufeinander zu gerichtet sind.

7. Vorrichtung zur Abgabe von Befestigungselementen nach Anspruch 1, worin das Befestigungselement (10) einen ersten Satz von Widerhaken (14), der sich vom distalen Ende des Körpers aus erstreckt, und einen zweiten Satz von Widerhaken (15) aufweist, der sich vom distalen Ende des Körpers axial versetzt vom ersten Widerhakensatz erstreckt; und wobei die Kanüle (20, 40, 60) so konfiguriert ist, dass sie zumindest einen Teil des Befestigungselements (10) aufnimmt und eine Vielzahl von Widerhaken (15) zurückhält, so dass sich die Vielzahl von Widerhaken vom Körper (11) in einem Winkel zur Längsachse erstreckt.

## Revendications

1. Dispositif de déploiement d'une agrafe, comprenant:
une agrafe (10) comprenant un corps (11), un axe longitudinal et une pluralité de barbes (14a, 15a) qui s'étendent à partir du corps le long de l'axe longitudinal; et
une canule (20, 40, 60), dans lequel la canule comprend une extrémité proximale (22, 42), une extrémité distale (21, 41), et une pointe distale (23, 43, 63), dans lequel l'extrémité proximale et l'extrémité distale de ladite canule sont chacune configurées de manière à recevoir une partie de l'agrafe, et la canule comporte une ouverture (24, 44) qui expose au moins deux de la pluralité de barbes (14a) lorsqu'au moins une partie de l'agrafe est disposée à l'intérieur de la canule, **caractérisé en ce que** l'ouverture est une fente qui s'étend à partir de la pointe distale en direction de l'extrémité proximale.

2. Dispositif de déploiement d'une agrafe selon la revendication 1, dans lequel la canule comporte une pointe distale tranchante (23, 43, 63).

3. Dispositif de déploiement d'une agrafe selon la revendication 1, dans lequel l'agrafe comporte une première pluralité de barbes (14) qui s'étendent à partir de l'extrémité distale du corps, et une deuxième pluralité de barbes (15) qui sont décalées de façon longitudinale et radiale par rapport au premier ensemble de barbes.

4. Dispositif de déploiement d'une agrafe selon la revendication 3, dans lequel le corps comprend une longueur dépourvue de barbes entre la première pluralité de barbes (14) et la deuxième pluralité de barbes (15).

5. Dispositif de déploiement d'une agrafe selon la revendication 3, dans lequel la première pluralité de barbes (14) s'étendent vers l'extérieur à partir du corps (11) dans une première direction qui est inférieure à 90 degrés par rapport à l'axe longitudinal, et la deuxième pluralité de barbes (15) s'étendent vers l'extérieur à partir du corps dans une deuxième direction qui est supérieure à 90 degrés et inférieure à 180 degrés par rapport à l'axe longitudinal.

6. Dispositif de déploiement d'une agrafe selon la revendication 5, dans lequel les pointes de la première pluralité de barbes (14) et les pointes de la deuxième pluralité de barbes (15) sont orientées les unes vers les autres.

7. Dispositif de déploiement d'une agrafe selon la revendication 1, dans lequel:
ladite agrafe (10) comprend un premier ensemble de barbes (14) qui s'étendent à partir de l'extrémité distale du corps, et un deuxième ensemble de barbes (15) qui s'étendent à partir de l'extrémité distale du corps en étant décalé axialement par rapport au premier ensemble de barbes; et
ladite canule (20, 40, 60) est configurée de manière à recevoir au moins une partie de l'agrafe (10) et à retenir une pluralité de barbes (15), de telle sorte que la pluralité de barbes s'étendent à partir du corps (11) à un certain angle par rapport à l'axe longitudinal.
